# EUROPEAN PATENT APPLICATION

(11) **EP 4 400 100 A1**
(43) Date of publication of application: **17.07.2024**
(21) Application number: 23382025.7
(22) Date of filing: 13.01.2023
(51) Int. Cl.: A61K 31/167, A61K 31/277, A61K 31/4166, A61K 31/4439, A61K 31/451, A61K 31/496, A61P 35/00

(54) **ANTIANDROGENS FOR USE IN THE TREATMENT OF CANCER**

(71) Applicant: Instituto de Investigación Biomédica de Salamanca De la Fundación Instituto Ciencia de la Salud De Castilla y León, 37007 Salamanca (ES); Universidad De Salamanca, 37008 Salamanca (ES)
(72) Inventor: GONZÁLEZ SARMIENTO, Rogelio, 37007 Salamanca (ES); HERRERO HERNÁNDEZ, Ana Belén, 37007 Salamanca (ES); ARROYO GARRAPUCHO, Nuria, 37007 Salamanca (ES)
(74) Representative: Hoffmann Eitle

(57) **Abstract**

The present invention refers to antiandrogens, particularly nonsteroidal antiandrogens, or salts derived thereof, for use in the treatment of cancer, wherein the cancer is characterized by the presence of androgen receptor-negative (AR-) tumor cells.

## Description

### FIELD OF THE INVENTION

The present invention refers to the medical field. Particularly, the present invention refers to antiandrogens, particularly nonsteroidal antiandrogens, or salts derived thereof, for use in the treatment of cancer, wherein the cancer is characterized by the presence of androgen receptor-negative (AR-) tumor cells.

### STATE OF THE ART

Breast cancer is the leading female cancer worldwide, both in terms of incidence and mortality. An estimated 2.3 million new cases were diagnosed in 2020, meaning that 1 in 8 cancers were identified as breast cancer. The high complexity of these tumours has meant that they are no longer considered a single disease. Broadly speaking, subtypes can be classified according to gene expression and the presence of different biomarkers. Molecular classification is vital for the selection of the therapeutic strategy, as the characteristics of each tumour determine the response to different treatments. Consequently, different therapeutic approaches are used. However, some patients develop resistance to such treatments after a variable period of time. In addition, some breast cancer subtypes, such as triple negative breast cancer, lack targeted therapies, so it is important to develop new strategies to treat them.

On the other hand, antiandrogens, also known as androgen antagonists or testosterone blockers, are a class of drugs that prevent androgens like testosterone and dihydrotestosterone (DHT) from mediating their biological effects in the body. They act by blocking the androgen receptor (AR) and/or inhibiting or suppressing androgen production. Nonsteroidal antiandrogens (NSAA), such as enzalutamide, are antiandrogens with a nonsteroidal chemical structure. They are typically selective and full or silent antagonists of the androgen receptor (AR) and act by directly blocking the effects of androgens like testosterone and dihydrotestosterone (DHT). NSAAs are used in the treatment of androgen-dependent conditions in men and women. They are the converse of steroidal antiandrogens (SAAs), which are antiandrogens that are steroids and are structurally related to testosterone. Particularly, enzalutamide is a second-generation antiandrogen drug commonly used in the treatment of metastatic prostate cancer as androgen deprivation therapy. This drug acts as an AR antagonist, preventing the binding of androgens such as testosterone or dihydrotestosterone, the translocation of AR from the cytoplasm to the nucleus and the binding of AR to DNA.

Such as it is indicated above, there is an unmet medical need of finding effective therapies aimed at treating specific cancer subtypes. The present invention is focused on solving this problem and an effective therapy for the treatment of cancer characterized by the presence of AR- tumor cells is herein provided.

### DESCRIPTION OF THE INVENTION

### Brief description of the invention

The present invention refers to antiandrogens, particularly nonsteroidal antiandrogens, or salts derived thereof, for use in the treatment of cancer, wherein the cancer is characterized by the presence of AR- tumor cells.

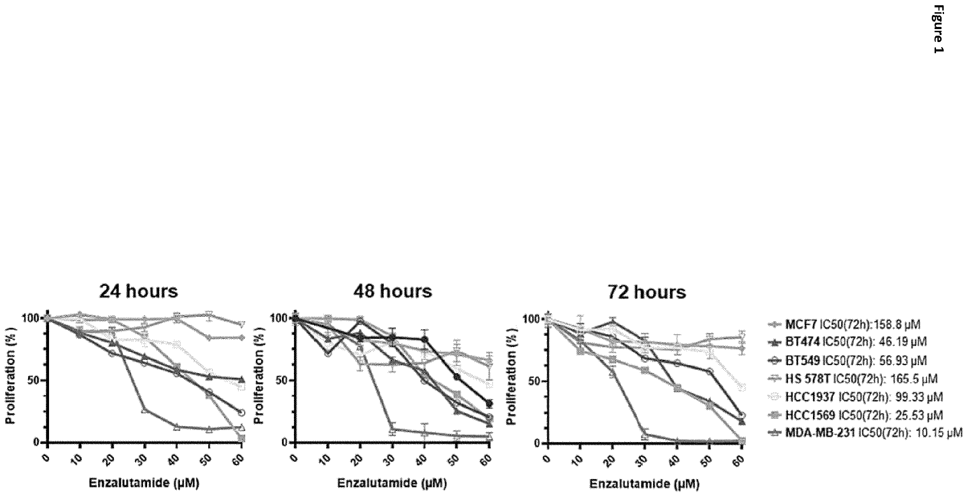

Enzalutamide has been used by the inventors of the present invention as proof of concept. Such as it is shown in the Examples provided below, the results show that enzalutamide inhibits AR- cell growth (see **Figures 1 to 4****).**

So, the first embodiment of the present invention refers to antiandrogens, or salts derived thereof, for use in the treatment of cancer, wherein the cancer is characterized by the presence of AR- tumor cells.

In a preferred embodiment, the antiandrogen is a nonsteroidal antiandrogen.

In a preferred embodiment, the cancer is selected from: breast cancer, ovarian cancer, glioblastoma or head and neck cancer.

In a preferred embodiment, the nonsteroidal antiandrogen is selected from: Enzalutamide, Aminoglutethimide, Apalutamide, Bicalutamide, Flutamide, Ketoconazole, Nilutamide or Topilutamide.

In a preferred embodiment, the antiandrogen is Enzalutamide.

In a preferred embodiment, the present invention refers to enzalutamide for use in the treatment of cancer, wherein the cancer is characterized by the presence of AR- tumor cells.

In a preferred embodiment, the present invention refers to enzalutamide for use in the treatment of cancer, wherein the cancer is characterized by the presence of AR- tumor cells and wherein the cancer is selected from: breast cancer, ovarian cancer, glioblastoma or head and neck cancer.

The present invention also refers to a method for treating cancer characterized by the presence of AR- tumor cells (for instance breast cancer, ovarian cancer, glioblastoma or head and neck cancer) which comprises administering to the patient a therapeutically effective amount or dose of an antiandrogen (for instance a nonsteroidal antiandrogen, such as for example: Enzalutamide, Aminoglutethimide, Apalutamide, Bicalutamide, Flutamide, Ketoconazole, Nilutamide or Topilutamide), or salt derived thereof, or a pharmaceutical composition comprising antiandrogens, or salts derived thereof, and pharmaceutically acceptable excipients or carriers.

The second embodiment of the present invention refers to a pharmaceutical composition comprising antiandrogens, or salts derived thereof, and pharmaceutically acceptable excipients or carriers, for use in the treatment of cancer, wherein the cancer is characterized by the presence of AR- tumor cells.

In a preferred embodiment, the pharmaceutical composition comprises nonsteroidal anti androgens.

In a preferred embodiment, the cancer is selected from: breast cancer, ovarian cancer, glioblastoma or head and neck cancer.

In a preferred embodiment, the pharmaceutical composition comprises: Enzalutamide, Aminoglutethimide, Apalutamide, Bicalutamide, Flutamide, Ketoconazole, Nilutamide or Topilutamide.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.
- By "therapeutically effective dose or amount" is intended an amount that, when administered as described herein, brings about a positive therapeutic response in a subject having cancer. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.
- "Pharmaceutically acceptable excipient or carrier" refers to an excipient that may optionally be included in the pharmaceutical composition of the invention and that causes no significant adverse toxicological effects to the patient.

### Description of the figures

**Figure 1****. Effect of enzalutamide on proliferation in breast cancer cell lines.** Study of cell proliferation by MTT assay in AR+ MCF7, BT474, BT549 and HS 578T lines and in AR-HCC1937, HCC1569 and MDA-MB-231 lines at 24, 48 and 72 hours after enzalutamide treatment. Error bars are not shown when smaller than the symbol size. IC50 (best fit and 95% confidence interval) shown below.

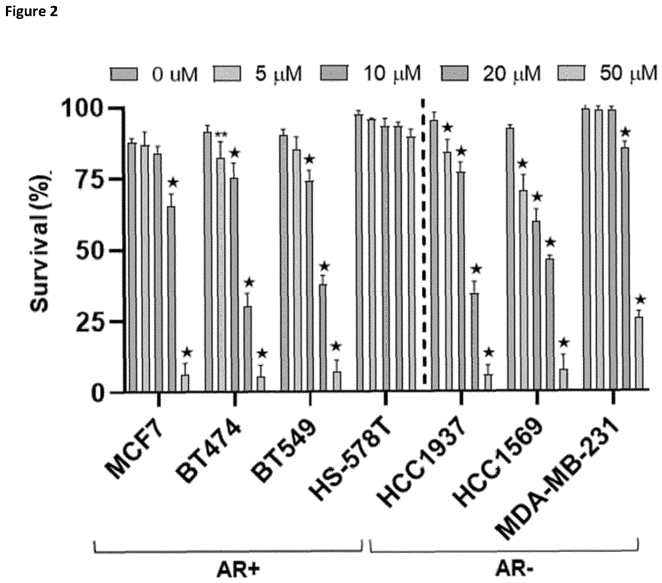

**Figure 2****. Effect of treatment with enzalutamide for 72 hours on apoptosis in breast cancer cell lines.** Cell viability study by Annexin V/propidium iodide labelling in AR+ MCF7, BT474, BT549 and HS 578T lines and in AR- HCC1937, HCC1569 and MDA-MB-231 lines after 72 hours of enzalutamide treatment. Error bars represent SD. Significance between C- and different conditions shown without brackets (^{∗}p < 0.05, ^{∗∗}p < 0.01, ★p<0.001).

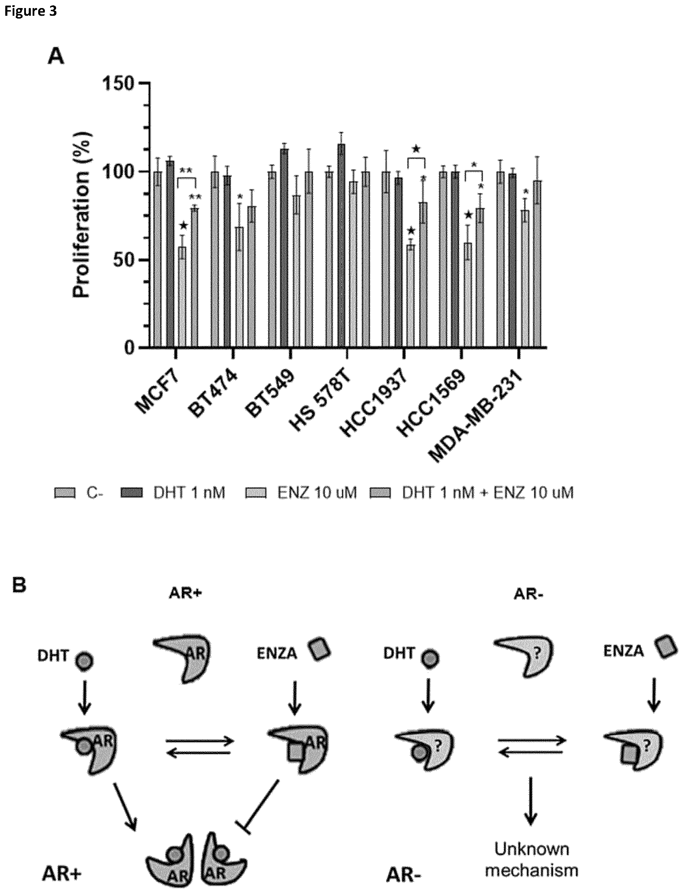

**Figure 3. A****)** Effect of 1 nM dihydrotestosterone, 10 µM enzalutamide and their combination on the indicated cell lines after 7 days of treatment. Data are the average of three independent experiments. Error bars represent SD. Statistically significant differences between C- and different conditions are shown without brackets (*p < 0.05, **p < 0.01, *p<0.001). **B)** Proposed mechanism of action of enzalutamide in AR- cells. In AR+ cells, DHT and enzalutamide compete for the AR active site. In the absence of enzalutamide, DHT binds reversibly to the AR, inducing conformational changes that lead to exposure of the nuclear localisation sequences, allowing transport of the AR to the nucleus and its dimerisation. In the presence of enzalutamide, this drug binds irreversibly to the AR, making it unable to activate transcription of target genes. In AR- cells, DHT may also compete with enzalutamide for an unknown common target that may also induce a survival response in the absence of enzalutamide that is abrogated in its presence.

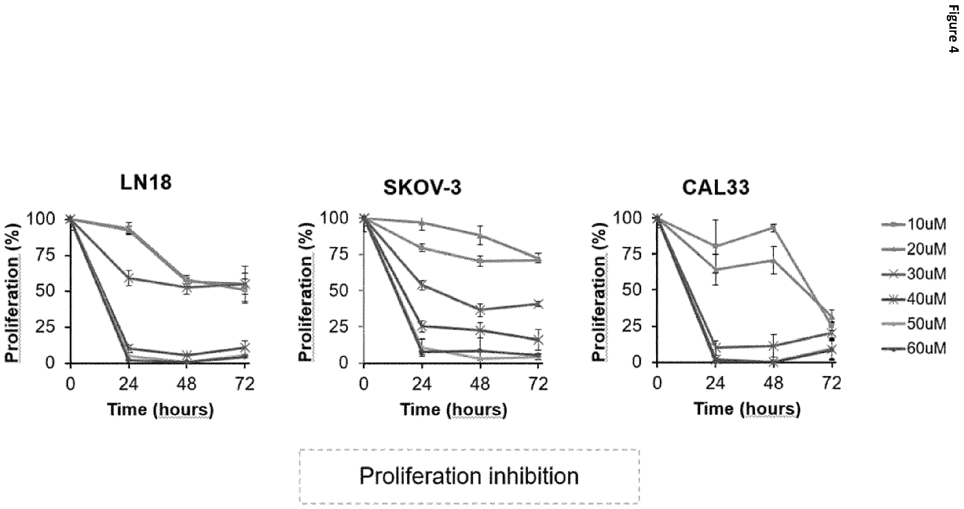

**Figure 4****. Effect of enzalutamide on proliferation in ovarian, glioblastoma and head and neck cancer cell lines.** Study of cell proliferation by MTT assay in the AR+ LN18 line and in the AR- SKOV-3 and CAL33 lines at 24, 48 and 72 hours after treatment with enzalutamide.

### Detailed description of the invention

The present invention is illustrated by means of the Examples set below without the intention of limiting its scope of protection.

### Example 1. Enzalutamide inhibits cell growth

Our results show that enzalutamide inhibits cell growth **(****Figure 1****).** After 24, 48 or 72 hours of treatment, the MDA-MB-231 (AR-) line was the most affected, followed by HCC1569 (AR-), BT474 (AR+) and BT549 (AR+), observing a dose-dependent effect. The same trend was observed 48 hours after treatment. Thus, the treatment inhibited cell proliferation in a dose- and time-dependent manner in all cell lines tested, reaching a higher efficacy at 72 hours.

### Example 2. Enzalutamide induced cell death in both AR+ and AR- lines

On the other hand, enzalutamide induced cell death in both AR+ and AR- lines **(****Figure 2****).** To determine whether the antiproliferative effect of enzalutamide was due to the induction of apoptosis, cell lines were treated with varying concentrations of the drug, stained with annexin V and propidium iodide and analysed by flow cytometry. We observed that enzalutamide induced apoptosis in a dose-dependent manner, independent of RA status.

### Example 3. Enzalutamide mechanism of action

To assess whether the effect of enzalutamide is androgen-independent, we tested the effect of enzalutamide on androgen-treated cancer cell lines. We studied the effects of dihydrotestosterone (DHT) (10 nM), enzalutamide (10 µM) and their combination on the different lines **(****Figure 3A****).** As for androgen stimulation, HS 578 T achieved the highest increase in cell growth, followed by BT549 and MCF7, both AR+. No significant differences were observed in BT474 (AR+) and AR- lines.

Enzalutamide treatment for 7 days induced a higher inhibition of proliferation than that observed at 72 hours. Interestingly, when enzalutamide was combined with DHT, the inhibition of cell proliferation was less than that of enzalutamide alone in AR+ and AR cells, even though some cell lines did not respond to growth stimulation with DHT. These results indicate that DHT might exert its effect on AR- cell lines either by decreasing enzalutamide binding to an unknown target or by modulating a transcriptional activity downstream of AR by an unknown mechanism. This hypothesis is depicted in **Figure 3B****.**

### Example 4. Enzalutamide effect is several types of solid tumors

Subsequently, we analysed whether the androgen receptor-independent mechanism of action of enzalutamide was specific to breast tumours or occurred in other tumours. We therefore studied whether there was an antiproliferative effect in cell lines of other types of solid tumours. We used the cell lines SKOV-3, an ovarian cancer line, LN18, from glioblastoma, and CAL33, derived from a head and neck tumour.

Our preliminary results indicate that, again, enzalutamide induces an inhibitory effect on proliferation regardless of the androgen receptor status of the tumours **(****Figure 4****).**

## Claims

1. Antiandrogens, or salts derived thereof, for use in the treatment of cancer, wherein the cancer is **characterized by** the presence of androgen receptor-negative (AR-) tumor cells.

2. Antiandrogens, or salts derived thereof, for use, according to claim 1, wherein the antiandrogen is a nonsteroidal antiandrogen.

3. Antiandrogens, or salts derived thereof, for use, according to any of the claims 1 or 2, wherein the cancer is selected from: breast cancer, ovarian cancer, glioblastoma or head and neck cancer.

4. Antiandrogens for use, according to any of the previous claims, wherein the nonsteroidal antiandrogen is selected from: Enzalutamide, Aminoglutethimide, Apalutamide, Bicalutamide, Flutamide, Ketoconazole, Nilutamide or Topilutamide.

5. Antiandrogens for use, according to any of the previous claims, wherein the nonsteroidal antiandrogen is Enzalutamide.

6. Enzalutamide for use, according to any of the previous claims, in the treatment of cancer, wherein the cancer is **characterized by** the presence of AR- tumor cells.

7. Enzalutamide for use, according to any of the previous claims, in the treatment of cancer, wherein the cancer is **characterized by** the presence of AR- tumor cells and wherein the cancer is selected from: breast cancer, ovarian cancer, glioblastoma or head and neck cancer.

8. Pharmaceutical composition comprising antiandrogens, or salts derived thereof, and pharmaceutically acceptable excipients or carriers, for use in the treatment of cancer, wherein the cancer is **characterized by** the presence of androgen receptor-negative (AR-) tumor cells.

9. Pharmaceutical composition, for use, according to claim 8, comprising nonsteroidal anti androgens.

10. Pharmaceutical composition, for use, according to claims 8 or 9, wherein the cancer is selected from: breast cancer, ovarian cancer, glioblastoma or head and neck cancer.

11. Pharmaceutical composition, for use, according to any of the claims 8 to 10, wherein the nonsteroidal antiandrogen is selected from: Enzalutamide, Aminoglutethimide, Apalutamide, Bicalutamide, Flutamide, Ketoconazole, Nilutamide or Topilutamide.

12. Pharmaceutical composition, for use, according to any of the claims 8 to 11, wherein the nonsteroidal antiandrogen is Enzalutamide.

13. Pharmaceutical composition comprising Enzalutamide, for use, according to any of the previous claims 8 to 12, in the treatment of cancer, wherein the cancer is **characterized by** the presence of AR- tumor cells.

14. Pharmaceutical composition comprising Enzalutamide, for use, according to any of the previous claims 8 to 13, in the treatment of cancer, wherein the cancer is **characterized by** the presence of AR- tumor cells and wherein the cancer is selected from: breast cancer, ovarian cancer, glioblastoma or head and neck cancer.
